(19) Europäisches Patentamt
European Patent Office
Office européen des brevets

(11) **EP 3 556 283 A1**

(12) **EUROPEAN PATENT APPLICATION**

(43) Date of publication:
**23.10.2019 Bulletin 2019/43**

(51) Int Cl.:
***A61B 5/0408*** *(2006.01)*   ***G01L 1/18*** *(2006.01)*

(21) Application number: **18167670.1**

(22) Date of filing: **17.04.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Centre National de la Recherche Scientifique 75016 Paris (FR)**
• **Université de Montpellier 34090 Montpellier (FR)**

(72) Inventors:
• **TODRI-SANIAL, Aida 34730 Prades-Le-Lez (FR)**
• **PANDEY, Reetu Raj 34090 Montpellier (FR)**
• **LIANG, Jie 34070 Montpellier (FR)**

(74) Representative: **IPAZ 18 rue de la République 34000 Montpellier (FR)**

(54) **PIEZOELECTRIC BIOSENSOR**

(57)     The present invention relates to a piezoelectric biosensor comprising carbon nanotubes (CNTs), optionally combined with an amplification system, to a process for preparing the same and to an apparatus comprising said piezoelectric biosensor, in particular a medical device usable for monitoring a body parameter.

A piezoresistive biosensor according to the invention comprises at least one micropattern of a composite mixture layered on a flexible support, said composite mixture comprising at least a flexible biocompatible polymer and carbon nanotubes (CNTs) and said at least one micropattern having a continuous rectangular shape.

**Figure 4**

**Description**

**DOMAIN OF THE INVENTION**

The present invention relates to a piezoelectric biosensor comprising carbon nanotubes (CNTs), optionally combined with an amplification system, to a process for preparing the same and to an apparatus comprising said piezoelectric biosensor, in particular a medical device usable for monitoring a biosignal or a body parameter.

**PRIOR ART**

Piezoresistive biosensors are gaining attention for biomedical application due to their ease of fabrication and their precise and fast response to biosignals from organs and tissues under small strain or compression. Conventional piezoresistive sensors are based on metallic or semiconductor strain gauges, their use is limited due to their incompatibility with biological tissues and organs. Among conductive nanomaterials, carbon nanotubes (CNTs) exhibit excellent thermal and electrical conductivity and have been associated with some polymers to develop biosensors.
US 8 850 897 discloses a flexible strip comprising rubber silicone and carbon nanotubes, and its use as a piezoresistive sensing.
US 9 625 330 discloses a pressure sensor comprising a mixture of CNTs and of PDMS. Xu and Allen ("Deformable strain sensors based on micropatterned MWCNTs/PDMS composites" J. Polymer Sci., part B: Polymer Physics, 2013, 51, 1505-12) discloses patterned strain sensors comprising PDMS-CNTs composites.
Lee et al. ("Self-adhesive epidermal carbon nanotube electronics for tether-free long-term continuous recording of biosignals" Nature Scientific Reports, 2014, 4, 6074) discloses a carbon-nanotube /PDMS electronic patch to measure specific electrical current at the skin, wherein said patch comprises a CNTs-PDMS layer combined with metal patterned poly-imide electrodes.
Franklin and Chen ("Length scaling of carbon nanotube transistors" Nature Nanotechnology Letters, 2010, 5, 858-862) discloses a carbon-nanotube field effect transistor having channel length from 3 $\mu$m to 15 nm.
So et al. ("Carbon nanotube based pressure sensor for flexible electronics" Nature Nanotechnology Letters, 2010, 5, 858-862) discloses a pressure sensor comprising vertically aligned carbon nanotubes supported by a PDMS matrix, integrated with field-effect transistor.
Koo et al. ("Wearable electrocardiogram monitor using carbon nanotube electronics and color-tunable organic light-emitting diodes" ACS NAno, 2017, 11, 10032-41) discloses an electrocardiogram device comprising four CNT transistors.
However, there is still a need for a sensitive, flexible and compact biosensor.
The inventors propose piezoresistive biosensor which is sensitive, accurate, compact and is made of a biocompatible polymer, it is responsive to thermal, mechanical and morphological changes. Said piezoresistive sensor comprises a micropattern providing optimum strain sensor sensitivity, through parameters such as the micropattern shape, the micropattern surface area, the amount of CNT in said micropattern and the porosity of polymer in micropattern to assure the optimum performance of the sensor. The inventors propose a process to compute micropattern shape and percolation threshold of CNTs by examining a "micropattern unit" area. The dimensions of micropattern unit area and CNT concentration are chosen such that to obtain the best strain sensitivity. The final sensor micropattern is then constructed by using "micropattern units" with optimal strain sensitivity parameter.
Due to its combined properties, a biosensor according to the invention presents a great interest in various applications requiring the detection of biosignals, in particular to be integrated in apparatuses for monitoring body parameters.
A sensor device of the invention can be integrated in an apparatus, particularly a medical apparatus, and allows continuous monitoring of weak body signal, for example by being put in contact with the skin, authorizing the activity and autonomy of a patient. Therefore, a sensor device according to the invention presents a great interest in various applications requiring the detection of signals, in particular to be integrated in apparatuses for monitoring body parameters.

**BRIEF DESCRIPTION OF THE INVENTION**

The present invention provides a piezoresistive biosensor comprising a micropattern of a composite mixture comprising a flexible polymer and carbon nanotubes (CNTs) layered on a flexible support, said micropattern having a continuous rectangular shape.
The present invention also provides a biosensor comprising a piezoresistive biosensor according to the invention connected to an amplification module, wherein both piezoresistive biosensor and amplification module are integrated on a same support.
The present invention further provides a process for designing the micropattern of a piezoresistive biosensor according to the invention, said process comprising a step of calculating the surface area of said micropattern.

The present invention further provides a process for preparing a piezoresistive biosensor and a biosensor comprising a piezoresistive biosensor connected to an amplification module, according to the invention.

The present invention further provides an apparatus comprising a piezoresistive biosensor or a biosensor according to the invention, and a process for detecting a biosignal using said apparatus.

Characteristics, advantages and uses of the subject matters of the present invention are more detailed hereunder, in an illustrated and non-limiting way. The disclosure of ranges expressed as "from ... to ..." mean that the limits are included in said ranges.

Figures 1A to 1C illustrate an example of micropattern design and the dimension and physical properties of micropattern units. Figure 1A represents a top view of a micropattern design, with "d" representing the dimension of a micropattern unit;
figure 1B represents a top view of a micropattern unit; figure 1C represents a 3D view of a micropattern unit.
Figure 2 illustrates the decreasing linear relationship between tunnelling resistance (ordinates) and single pattern area and total sensor area (abscissa).
Figures 3A and 3B illustrate tunnelling resistance between carbon nanotubes, wherein arrows indicate said tunnelling; figure 3A and 3B respectively show tunnelling resistance between carbon nanotubes in the absence and in the presence of stress or strain.
Figure 4 represents a schematic top view of an example of a piezoresistive biosensor, "d" representing the dimension of a micropattern unit; on the left and right of the micropattern the two electrodes are represented as grey rectangles, their dimensions (width and length) are 4d and 15d, respectively.
Figures 5A to 5C represent a piezoresistive biosensor according to example 1, figure 5A is a side view, figure 5B a top view and figure 5C a detail of the micropattern of said biosensor.
Figure 6 represents the connection between a piezoresistive sensor according to the invention and an amplification device, in a device according to example 3.
Figures 7A and 7B represent a piezoresistive biosensor according to example 2, figure 7A is a side view and figure 7B a top view of said biosensor.

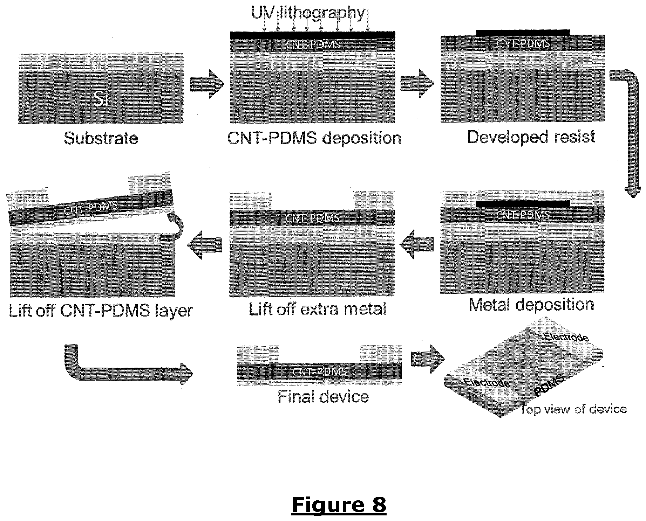

Figure 8 represents the main steps of a process for preparing a piezoresistive biosensor according to the invention.
Figure 9 represents the main steps of a process for preparing a sensor device according to the invention.

For ease of illustration, the various figures are not drawn to scale.

## DETAILED DESCRIPTION OF THE INVENTION

In a first aspect, the present invention relates to a piezoresistive biosensor comprising at least one micropattern of a composite mixture layered on a flexible support, wherein said composite mixture comprises at least a flexible biocompatible polymer and carbon nanotubes (CNTs) and wherein said at least one micropattern has a continuous rectangular shape.
The term "biosensor" refers to an analytical device comprising a physico-chemical detector able to detect, and preferably to quantify, a biosignal, wherein a "biosignal" is defined as a signal emitted by a living body, organ, or tissue. "Piezoresistive biosensor" refers to a biosensor able to detect a piezoresistive effect, which is a change in the electrical resistivity of said biosensor when mechanical strains are applied. Therefore, a piezoresistive biosensor according to the present invention may be defined as a sensor of a mechanical signal, and particularly as a strain biosensor. A "flexible support" and a "flexible polymer" are able to curve or bend without breaking, said flexible support or polymer are also stretchable as they do not break when submitted to strain.
A "composite mixture" refers to a combination of different substances. A composite mixture of a micropattern in a piezoresistive biosensor according to the invention comprises at least:

- a synthetic biocompatible polymer, defined as a large molecule composed of many covalently bonded repeated subunits, wherein a "biocompatible" material is able to interact with a living body, including humans and animals, without representing any toxicity for said living body,
- and carbon nanotubes (CNTs), defined as allotropes of carbon with a cylindrical nanostructure ; carbon nanotubes are classified as single-walled nanotubes (SWNTs) and multi-walled nanotubes (MWNTs).

In said composite mixture, CNTs and polymer are homogenously mixed.

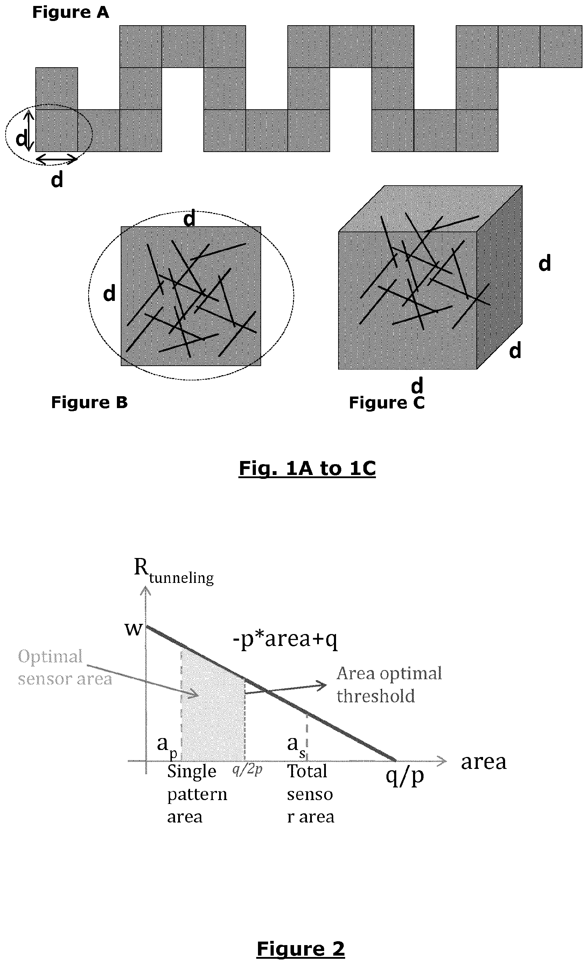

A "micropattern" refers to a miniaturized pattern. In a biosensor according to the invention, said micropattern is defined by its continuous rectangular shape, wherein said shape is curved forming right angles, as exemplified in figures 1A to 1C. Said micropattern is also defined by its dimensions, including its shape and thickness. The design of said micropattern is obtainable by a process such as disclosed in the present application.

A biosensor according to the invention contacts with the surface of a living body, for example skin, through said micropattern, which thus plays a critical role in sensor performance. The micropattern is designed to provide optimum sensitivity to the biosensor, its design includes the following parameters: i) micropattern surface area, ii) micropattern shape, iii) CNTs concentration and iv) physico-chemical properties of the polymer in the composite mixture.

Micropattern unit

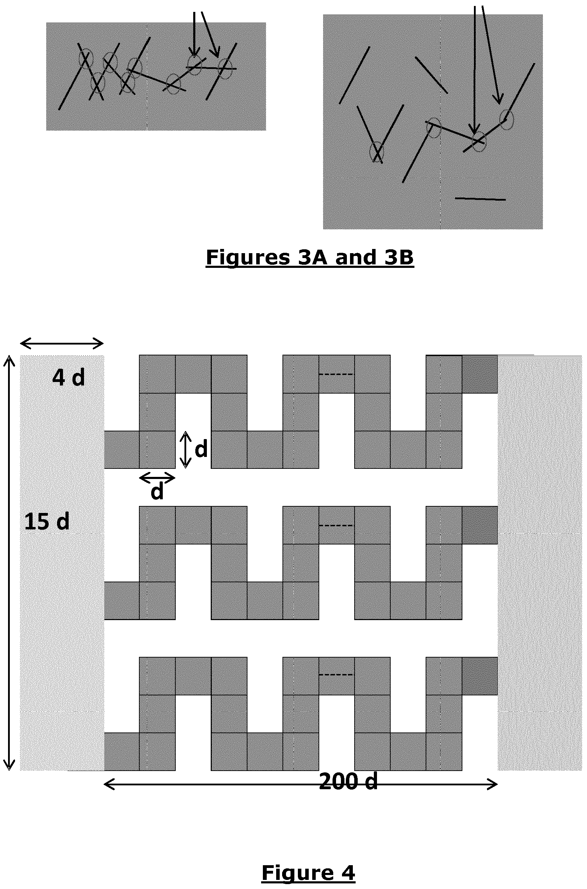

In a particular embodiment, a micropattern of a piezoresistive biosensor according to the present invention is made of a composite mixture comprising a flexible polymer and CNTs, wherein the percentage of CNTs, as expressed as a percentage of weight of said polymer, ranges from 0.0001 to 2.5 %, from 0.001 to 2.4 %, from 0.01 to 2.2 %, from 0.01 to 2.0 %, from 0.015 to 2.5 %, from 0.02 to 2%, from 0.1 to 1.5 %, from 0.2 to 1.5 % or from 0.3 to 1 %. The range of 0.0001 to 2.5 wt% CNTs corresponds to 0.001 to 25.9 g/L, respectively. The proportion of CNTs within the composite mixture is important because, when a strain is applied, CNTs get closer together to create a network, or disconnect from each other due to stretching, as illustrated in figures 2, 3A and 3B. Connection or disconnection of CNTs changes the piezoresistivity of the polymer-CNTs composite and ultimately the sensor sensitivity. Said sensitivity can be explained by the percolation theory. The concentration of CNTs is comprised between a minimum value, wherein CNT would cluster and segregate from each other, and is inferior to a maximum value, wherein CNT would remain connected, therefore leading to an undetectable change in piezoresistance when strain is applied.

In a particular embodiment, in a composite mixture of a piezoresistive biosensor according to the present invention, carbon nanotubes are Multi-Walled Carbon Nanotubes (MWCNTs).

In another particular embodiment, a micropattern of a piezoresistive biosensor according to the present invention comprises at least one repetition of a design having a rectangular shape fluctuation. The thickness of said micropattern is from 10 to 100 $\mu$m, from 15 to 50 $\mu$m and preferably of 25 $\mu$m.

In a particular embodiment, a piezoresistive biosensor according to the present invention comprises one, two, three or four micropatterns in a parallel circuit.

In a particular embodiment, a piezoresistive biosensor according to the present invention comprises at least one micropattern, wherein the total surface area of said at least one micropattern (i.e. the total micropatterned surface of said biosensor) represents from 35 % to 65 %, from 40 % to 60 %, from 45 % to 55 %, and preferably 50 % of the surface area of said piezoresistive biosensor. The surface area of said piezoresistive biosensor is the surface area of the flexible polymer support, which does not comprise the surface area of the electrodes.

In another particular embodiment, a micropattern of a piezoresistive biosensor according to the present invention has an optimal surface area which is defined as:

$$area = {}^{q}\!/_{2p} - a_p$$

wherein p is the ratio of composite resistance versus sensor area ; q is the cross point of the linear relationship of composite resistance and sensor area; $a_p$ is the area of one micropattern.

A biosensor according to the invention comprises a micropattern which is designed by balancing the concentration of CNTs in the composite mixture and the micropatterned surface area. Said micropattern is designed according to a process of the invention in order to provide a maximum contact to skin while using the minimal CNTs concentration, such that optimal performance can be attained by percolation of CNTs.

In a particular embodiment of a piezoresistive biosensor according to the invention, said composite mixture comprises a flexible biocompatible polymer, wherein said polymer can be present as a homopolymer, containing only a single type of repeated units, or as a copolymer, containing two or more types of repeated units. Said polymer is preferably compatible with carbon nanotubes as it preserves CNTs intrinsic conductive properties.

In a particular embodiment, a piezoresistive biosensor according to the present invention comprises a composite mixture comprising a flexible biocompatible polymer chosen among:

- siloxane, preferably PolyDimethylSiloxane (PDMS, dimethicone, -[O-Si(CH$_3$)$_2$]$_n$),
- acrylate, preferably Poly(methylmetacrylate) (PMMA),
- polycarbonate and
- poly-L-lactide (PLA).

A polymer comprised in said micropattern is also characterised by its characteristics of: susceptibility to degradation and permeability.

Biosensor is a « strain biosensor », is flexible, wearable.

In a more particular embodiment, a piezoresistive biosensor according to the present invention comprises a composite mixture comprising siloxane and CNTs, preferably comprising PDMS and MWCNTs.

In another particular embodiment, a piezoresistive biosensor according to the present invention comprises a support comprising a flexible polymer chosen among :

- siloxane, preferably PolyDimethylSiloxane (PDMS),
- acrylate, preferably Poly(méthylmetacrylate) (PMMA),
- polycarbonate and
- poly-L-lactide (PLA).

In an embodiment of the present invention, the polymers comprised in the micropattern and in the support are identical In another embodiment of the present invention, the polymers comprised in the micropattern and in the support are different.

In a particular embodiment of a piezoresistive biosensor according to the present invention, said micropattern comprises a polymer which porosity is adapted to the conductivity of said micropattern. Porosity, or void fraction, is a measure of the empty spaces in a material, and is a fraction of the volume of voids over the total volume, between 0 and 1, or as a percentage between 0% and 100%. In a particular embodiment of a piezoresistive biosensor according to the present invention, said micropattern comprises a polymer which porosity ranges from 0.45 to 0.6, or from 45% to 60%.

In a particular embodiment, a composite mixture in a piezoresistive biosensor according to the invention comprises a polymer, CNTs and at least one additive, wherein said additive may represent less than 3 %, less than 2 %, less than 1.5 %, less than 1 % or less than 0.5 %, as expressed by weight of polymer.

As an example of additives, plasticisers, which may increase polymers flexibility, may be present.

In a particular embodiment, a composite mixture of a piezoresistive sensor according to the invention also comprises a product designed as a « filler », which is added to the mixture, in proportions easily determined by a person of the art, in order to replace a part of said polymer by a less expensive material.

Preferably, in a piezoresistive biosensor according to the invention, the micropattern is connected to an electrical interface, said electrical interface comprising at least one terminal element at one extremity of said micropattern and at least one terminal element at the other extremity of said micropattern. Said electrical interface preferably consists of a pair of electrodes. According to a particular embodiment, said electrodes are chosen among the following: Cr/Au, Pd/Au, Cr/Pt and Pd/Pt electrodes.

In a second aspect, the present invention concerns a biosensor device comprising:

- a piezoresistive biosensor according to the invention, and
- an amplification module comprising at least an organic field effect transistor (FET).

In a particular embodiment of said second aspect, the present invention concerns a biosensor device comprising:

- a piezoresistive biosensor according to the invention, and
- an amplification module comprising at least one organic field effect transistor (FET),

wherein said piezoresistive biosensor and said amplification system are co-integrated on a same support.

In a particular embodiment, a biosensor device according to the present invention comprises several organic FETs in a parallel circuit, more particularly said sensor device comprises 2, 3, 4, 5, 6 or more organic FET. In a preferred embodiment, a sensor device according to the present invention comprises 4 organic FETs in a parallel circuit.

In a particular embodiment, a sensor device according to the present invention comprises several carbon nanotubes FETs (CNTFETs) in a parallel circuit, more particularly said sensor device comprises 2, 3, 4, 5, 6 or more CNTFETs. In a preferred embodiment, a sensor device according to the present invention comprises 4 CNTFETs in a parallel circuit.

In another particular embodiment, a sensor device according to the present invention comprises several organic FETs in a parallel circuit and a carbon nanotubes (CNTs) sensor device.

In a more particular embodiment, a sensor device according to the present invention comprises several organic FETs in a parallel circuit and a carbon nanotubes (CNTs) sensor device, wherein said sensor module and said amplification module are co-integrated on a same flexible support. In a more particular embodiment, a sensor device according to the present invention comprises several carbon nanotubes FETs (CNTFETs) in a parallel circuit and a carbon nanotubes (CNTs) sensor device, wherein said sensor module and said amplification module are co-integrated on a same flexible support.

A flexible support is able to curve or bend without breaking, said flexible support is preferably also stretchable, as it does not break when submitted to a strain. In a biosensor device according to the invention, the support exhibits at least one of the following properties: flexibility, stretchability, biocompatibility, permeability, wearability by a living, such as a human, an animal or a vegetal organism.

In a particular embodiment, a sensor device according to the present invention comprises several carbon nanotubes FETs (CNTFETs) in a parallel circuit and a carbon nanotubes (CNTs) sensor device, wherein said sensor module and said amplification module are co-integrated on a same flexible support, wherein said support comprises, or consists of, a flexible and biocompatible material.

In a more particular embodiment, a sensor device according to the present invention comprises at least one organic FET, said organic FET having a channel length from 1 to 3 $\mu$m, preferably a channel length of 2 $\mu$m.

In another particular aspect, a biosensor device of the invention comprises several organic FET, and preferably comprises one, two, three, four or five FETs, preferably four FET.

In a third aspect, the present invention concerns a process for preparing a piezoresistive biosensor according to the invention, said process comprising the steps of:

- providing a flexible support, and
- forming at least one micropattern of a composite mixture layered on said flexible support, said composite mixture comprising at least a flexible biocompatible polymer and CNTs, wherein said micropattern has a continuous rectangular shape.

A micropattern of a sensor device according to this aspect may be formed by any technique known by a person skilled in the art, and particularly by lithography or printing, including screen print.

In a particular embodiment, a process for preparing a piezoresistive biosensor according to the invention comprises a step of calculating the optimal surface area of the micropattern with respect to the surface area of the piezoresistive biosensor by using the following formulae:

$$area\% = \frac{q/2p - a_p}{a_s - a_p} \cdot 100$$

wherein p is the ratio of composite resistance versus sensor area ; q is the cross point of the linear relationship of composite resistance and sensor area; $a_p$ is the area of one micropattern, and $a_s$ is the total sensor area.

In a micropattern of a piezoresistive biosensor according to the invention, a "micropattern unit" is defined as a volume having dimensions d x d x d, as exemplified in figures 1A to 1C. For a single "micropattern unit", the conductive network of MWCNTs on the composite CNTs/polymer, and for example a composite MWCNT/PDMS, can be understood by using the percolation theory. Uniform distribution of CNTs within the composite is desired, however, in practice there are not straight/aligned CNTs but rather tangled CNT lines as in Figures 1B and 1C. In considering the formation of a percolation network, the distribution and connections of CNTs is highly important. The aspect ratio (length/diameter) of CNTs is taken into account and has an impact of quantum tunneling resistance between CNT-to-CNT connections. For a single micropattern unit, the percolation threshold is calculated in terms of weight percentage as:

$$wt\% = \frac{\rho_{CNT} \times V_{CNT}}{\rho_{CNT} \times V_{CNT} + \rho_{PDMS} \times V_{PDMS}} \qquad \text{(Equation 1)}$$

where $\rho_{CNT}$ and $\rho_{PDMS}$ are the concentration densities of CNTs and PDMS, respectively. Volume of CNTs ($V_{CNT}$) depends on the total number of CNTs and dispersion quality. Volume of PDMS ($V_{PDMS}$) depends on the volume of micropattern unit ($dxdxd$). The $wt\%$ gives the percolation threshold (or minimum concentration of CNTs) for micropattern unit area. It is important to note that the $\rho_{PDMS}$ density of PDMS (such as porosity) also matters on the CNT percolation threshold and can be an essential feature for reducing $wt\%$.

By using percolation theory, the composite material conductivity ($\sigma$) of the micropattern unit is calculated as:

$$\sigma = \sigma_0(wt_{max} - wt_{CNT})^t \qquad \text{(Equation 2)}$$

where $\sigma_0$ is a constant, $wt_{max}$ is the max weight fraction (for example 2.5%) of CNTs, and $wt_{CNT}$ is weight fraction computed in EQ1, and t is the critical exponent based on percolation.

Then, the sensitivity of the sensor (Gauge Factor (GF)) is calculated by taking the ratio of the resistance change to

lengthwise strain $\varepsilon$, as:

$$S = \frac{\Delta R / R_0}{\varepsilon} \qquad \text{(Equation 3)}$$

Thus, the micropattern shape is designed by engineering the resistivity of each micropattern unit. By understanding the resistivity change on the micropattern unit, a micropattern shape that will lead to optimal performance of strain sensor can be designed in a controllable manner. Combining micropattern units in a regular and continuous pattern allows continuity of CNT networks with controllable sensitivity performance.

For a given sensor area, the micropatterns are distributed on *area%* of sensor area as shown in figure 2.

The following analytical steps are performed to compute the *area%* for patterns to use in the total sensor area:

a) Sensitivity is defined as a function of sensor area

$$S(area) = \frac{\Delta R / R_0 / area}{\varepsilon} = \frac{\Delta R}{\varepsilon R_0} \cdot area = \frac{R_{tunneling}(area)}{\varepsilon R_0} \cdot area \qquad \text{(Equation 4)}$$

the change on resistance $\Delta R$ on MWCNT/PDMS due to the tunneling resistance $R_{tunneling}(area)$ due to CNT tubes agglomeration and creating resistive paths is expressed.

b) Tunneling resistance with area

For a given concentration of CNTs (*wt%*) and for a given stress $\varepsilon$, the relationship between tunneling resistance and area is approximated as in Figure 2, which shows the decreasing linear relationship between tunneling resistance and area. Figures 3A and 3B illustrate how tunneling resistance changes with area for a fixed concentration CNTs and a given stress.

Analytically the sensitivity can be expressed with respect to area and tunneling resistance as:

$$S(area) = \frac{R_{tunneling}(area)}{\varepsilon R_0} \cdot area = \frac{(-p \cdot area + q)}{\varepsilon R_0} \cdot area = \frac{-p \cdot area^2 + q \cdot area}{\varepsilon R_0} \qquad \text{(Equation 5)}$$

by taking first order derivative, the critical point or the optimal surface area can be identified as:

$$\frac{dS(area)}{d(area)} = -2p \cdot area + q = 0 \qquad \text{(Equation 6)}$$

where optimal area threshold is found as *area = q/2p.*

For any given single pattern shape, its area can be defined as $a_p$ and the total sensor area can be defined as $a_s$.

To calculate the optimal pattern area for any given sensor with parameters $a_p$ and $a_s$ (as in Figure 2), the optimal area is as:

$$area\% = \frac{q/2p - a_p}{a_s - a_p} \cdot 100 \qquad \text{(Equation 7)}$$

In an example of a sensor design area with micropattern unit of d, the area% is 50%.

In a fourth aspect, the present invention relates to a process for preparing a piezoresistive biosensor according to the invention, comprising designing a micropattern which surface area is calculated by a process according the third aspect of the invention, and comprising the successive steps of:

- providing a substrate,
- preparing a composite by mixing a solution of a flexible biocompatible polymer and a solution of CNTs,
- depositing a layer of said composite on the surface of the substrate,
- patterning said composite in at least one micropattern as designed in the first step,

- forming metallic electrodes by depositing metal on the opposite ends of said patterned composite layer, and
- removing said substrate, to obtain said piezoresistive biosensor.

In a fifth aspect, the present invention relates to a process for preparing a biosensor according to the invention, said biosensor comprising:

- a piezoresistive biosensor
- an amplification system comprising at least an organic filed effect transistor (FET),

wherein said piezoresistive biosensor and amplification systems are integrated on a same substrate, wherein said process comprises designing a micropattern which surface area is calculated by a process according the third aspect of the invention, and the successive steps of :

- providing a substrate,
- depositing a first layer of a first polymer on a substrate,
- depositing a protective material on a first part of the surface of said first polymer layer,
- depositing a second layer of said first polymer on said protective material and on said first layer of said first polymer,
- removing said protective material and the first part of said second layer of said first polymer,
- depositing a protective material on the second part of said first polymer layer,
- preparing a composite mixture by mixing a solution of a second polymer and a solution of CNTs, wherein said solutions of polymer and said CNTs are homogeneously mixed,
- depositing a layer of said composite mixture on the surface of said first polymer layer,
- patterning said composite in at least one micropattern,
- removing said protective light sensitive material,
- forming metallic electrodes by depositing metal on the opposite extremities of said patterned composite layer, and
- depositing of organic FET channel material, by an adapted method, such as for example spin coating, screen printing, thermal evaporation or vacuum deposition, more particularly, depositing a liquid solution of CNTs, then drying said CNTs solution,
- if said FET channel material is CNTs: aligning CNTs, preferably by dielectrophoresis, and depositing to gate metal,
- removing said substrate, to obtain said sensor device.

The substrate is easily chosen among known substrates by a person skilled in the art, and is preferably made of one of the following: silicon, SiO2, GaN and Germanium.

A micropattern of a sensor device may be formed by any technique known by a person skilled in the art, and particularly by lithography or printing, including screen print.

In a fifth aspect, the present invention relates to an device comprising at least one piezoresistive biosensor according to the invention, or at least one piezoresistive biosensor obtained by a process according the invention, wherein said device is a biosignal monitoring device chosen among a non-implantable device and an implantable device.

A device according to the invention is for example a patch which may be held again the skin of a human or an animal.

The present invention further relates to the use of at least one device according to the invention:

- for providing a biosignal chosen among: electroencephalogram (EEG), electrocardiogram (ECG), electromyogram (EMG), mechanomyogram (MMG), electrooculography (EOG), galvanic skin response (GSR), magnetoencephalogram (MEG), or
- for monitoring a body parameter chosen among: respiratory flow, body temperature, heart rate and blood pressure.

As an example, providing an electrocardiogram requires the presence of preferably 3 devices according to the invention.

The present invention further relates to a process for providing a biosignal or for monitoring a body parameter comprising the steps of:

- attaching at least one device according to the invention to a surface of said body,
- passing a current through said at least one device, and
- sensing the current passing through said at least one device.

Said process may be used for the diagnosis of disease or monitoring of health.

## EXAMPLES

### Example 1: Process for preparing a piezoresistive biosensor

#### 1.1. Material and methods

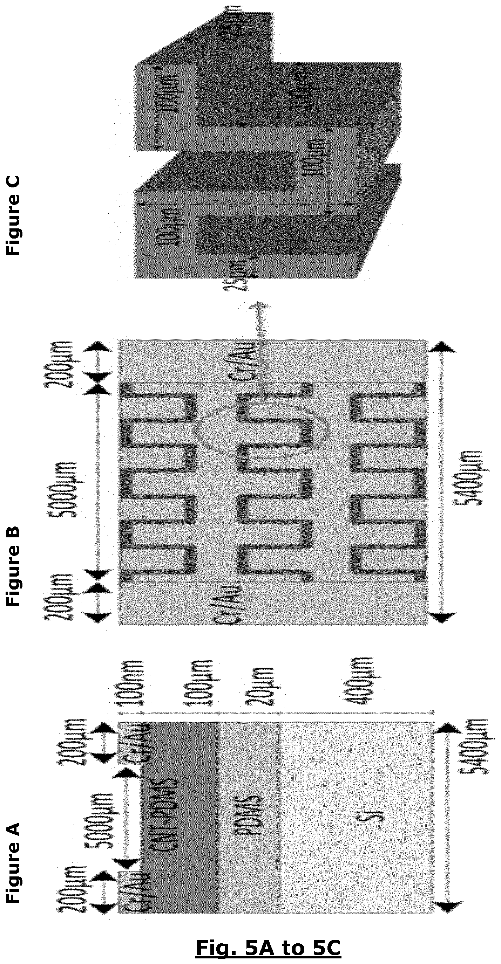

An optimized micropattern is defined as described in the present application. The calculated surface covered by the micropattern represents 50% of the surface of the PDMS layer. Said micropattern has a rectangular shape as shown in figure 5C.

A piezoresistive biosensor according to figure 5A to 5C is prepared as follows:

- a PDMS layer is deposited on a silicon substrate,
- a composite comprising PDMS and MWCNT is prepared by mixing homogeneously a solution of PDMS and a solution of CNTs, wherein CNTs are present in proportions varying from 0.0001 % to 2.5 %, as expressed as a percentage of the volume of the PDMS solution,
- a layer of said composite is deposited by spin-coating on said PDMS layer,
- PDMS-CNT composite is patterned by UV lithography, according to a predetermined micropattern as calculated, as illustrated in figure 4,
- metallic electrodes are deposited on said layer of composite,
- extra metal is lifted,
- the silicon substrate is removed from the biosensor.

#### 1.2. Results

A piezoresistive biosensor comprising a micropattern of a composite mixture comprising PDMS and MWCNTs is prepared as described in 1.1, wherein said micropattern is defined as follows:

- Micropattern unit : d
- Rectangular shape comprising n repetitions of a rectangular shape fluctuation consisting of p micropattern units characterized by a dimension d (or : comprising x micropattern units in a shape defined as ...)
- Three micropatterns on the surface
- Distance between the two electrodes : 200d
- Electrode dimensions : 4d x 15d

A piezoresistive biosensor prepared as described in 1.1. has the following characteristics :

Micropattern thickness: 25 $\mu$m (micron)
Resistivity = around 0.4KOhm-5KOhm per square
Flexibility= for CNT-PDMS composite comprising at most 2.5% weight and small strain from heart, the Young Modulus is comprised between 2 to 2.5MPa
Conductivity = related to resistivity (1/resistivity)

Said piezoresistive biosensor is able to be integrated in a device for the detection of a body parameter, such as respiratory flow, body temperature, heart rate and blood pressure.

### Example 2: Piezoresistive biosensor combined with a CNTFET

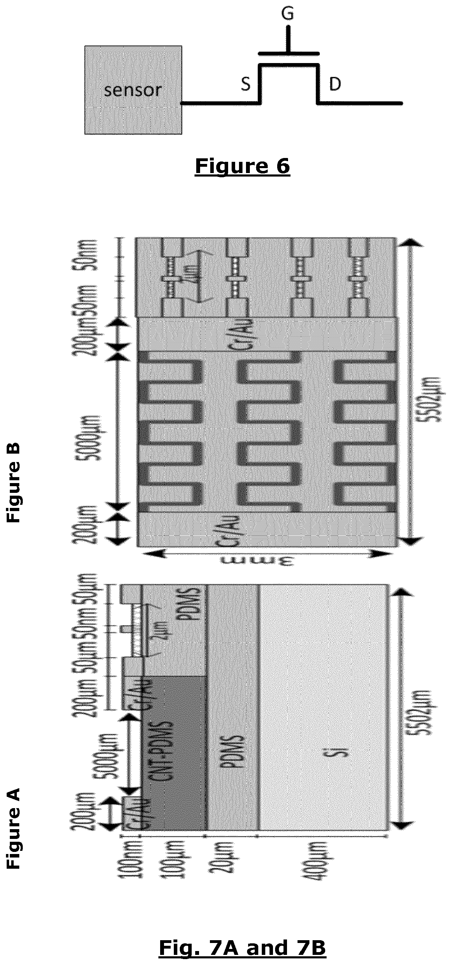

For health monitoring applications, electrocardiogram signal is a weak signal of about 1 mV. It is important to amplify said weak signal obtained from a sensor to detect the output without any signal loss before transmitting it to signal processing units, such as an operational amplifier.
A carbon nanotube filed effect transistor (CNTFET) is connected directly with the sensor output and enhances the signal received from the sensor, as represented on figure 6. The sensor output is connected to the source of CNTFET (p-type) having a gate length of 2 $\mu$m. The CNTFET gate is modulated by external voltage. The output is on the drain terminal of the CNTFET.
In a device according to example 2, one p-type CNTFET which can be modulated by top-gate is co-integrated next to the biosensor and on the same substrate, to amplify the weak signal emitted by said sensor, as represented on figure 7A. The CNTFET is integrated on the same flexible support as the biosensor, without the need of any wire bonding.
When preparing a sensor device comprising, as a sensor module, a piezoresistive biosensor comprising at least one micropattern of a composite mixture, the optimal surface area of the micropattern is calculated with respect to the surface area of the piezoresistive biosensor by using the following formulae:

$$area\% = \frac{q/2p - a_p}{a_s - a_p} \cdot 100$$

wherein p is the ratio of composite resistance versus sensor area ; q is the cross point of the linear relationship of composite resistance and sensor area; $a_p$ is the area of one micropattern, and $a_s$ is the total sensor area.

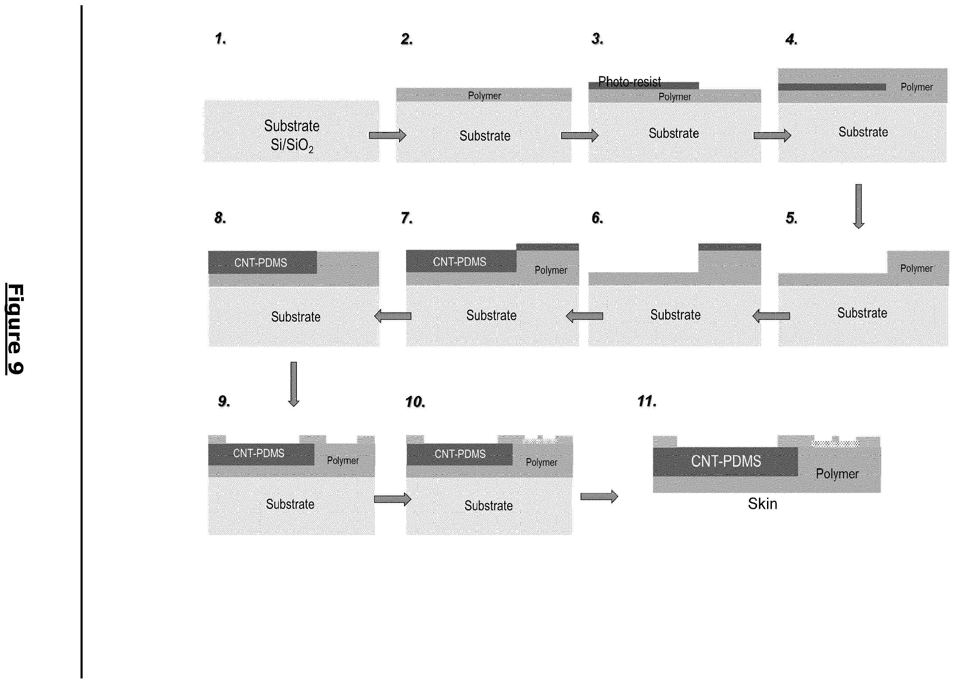

A piezoresistive biosensor according to figure 7A and 7B is prepared as follows (numbers refer to the successive steps indicated in figure 9):

- providing a Si/SiO2 substrate (1)
- depositing a first layer of a first polymer, for example PDMS, on said substrate (2)
- depositing a protective material on a first part of the surface of said first polymer layer, said protective material being for example a photoresist (3),
- depositing a second layer of said first polymer on said protective material and on said first layer of said first polymer (4),
- removing said protective material and the first part of said second layer of said first polymer (5),
- depositing a protective material on the second part of said first polymer layer (6),
- preparing a composite mixture by mixing a solution of a second polymer, for example PDMS, and a solution of CNTs,
- depositing a layer of said composite mixture on the surface of said first polymer layer (7)
- patterning said composite in at least one micropattern as calculated in the first step of the process (8),
- removing said protective light sensitive material (8),
- forming metallic electrodes by depositing metal on the opposite extremities of said patterned composite layer (9),
- depositing a liquid solution of CNTs, then drying said CNTs solution (10), on the polymer surface area corresponding to the at least one organic FET
- aligning CNTs, preferably by dielectrophoresis, and depositing the gate metal (11),
- removing said substrate, to obtain said piezoresistive biosensor.

Step 11 also represents the contact, for example, with skin.

**Claims**

1. Piezoresistive biosensor comprising at least one micropattern of a composite mixture layered on a flexible support, wherein said composite mixture comprises at least a flexible biocompatible polymer and carbon nanotubes (CNTs) and wherein said at least one micropattern has a continuous rectangular shape.

2. Piezoresistive biosensor according to the preceding claim, wherein said CNTs represent from 0.0001 % to 2.5 % by weight of said polymer.

3. Piezoresistive biosensor according to any of the preceding claims, wherein said CNTs are Multi-Walled Carbon Nanotubes (MWCNTs).

4. Piezoresistive biosensor according to any of the preceding claims, wherein said at least one micropattern comprises at least one repetition of a design having a rectangular shape fluctuation.

5. Piezoresistive biosensor according to any of the preceding claims, wherein said piezoresistive biosensor comprises one, two, three or four micropatterns in a parallel circuit.

6. Piezoresistive biosensor according to any of the preceding claims, wherein the surface area of the micropattern represents from 35 % to 65 %, from 40 % to 60 %, from 45 % to 55 %, and preferably 50 % of the surface area of said piezoresistive biosensor.

7. Piezoresistive biosensor according to anyone of the preceding claims, wherein the optimal surface area of the micropattern is defined as:

$$area = {}^{q}/_{2p} - a_p$$

wherein p is the ratio of composite resistance versus sensor area ; q is the cross point of the linear relationship of composite resistance and sensor area; $a_p$ is the area of one micropattern.

8. Piezoresistive biosensor according to any of the preceding claims, wherein said biocompatible flexible polymer is chosen among :

- siloxane, preferably PolyDimethylSiloxane (PDMS),
- acrylate, preferably Poly(méthylmetacrylate) (PMMA),
- polycarbonate and
- poly-L-lactide (PLA).

9. Piezoresistive biosensor according to any of the preceding claims, wherein the micropattern is connected to an electrical interface, said electrical interface comprising at least one terminal element at one extremity of said micropattern and at least one terminal element at the other extremity of said micropattern.

10. Biosensor device comprising:

- a piezoresistive biosensor according to any of the preceding claims, and
- an amplification module comprising at least one organic field effect transistor (FET).

11. Biosensor device according to the preceding claim, wherein the piezoresistive biosensor and the amplification system are co-integrated on a same support.

12. Biosensor device according to any of the preceding claims, wherein the organic FET is a carbon nanotubes FET (CNTFET).

13. Process for preparing a piezoresistive biosensor according to any of claims 1 to 9, comprising the steps of :

- providing a flexible support, and
- forming at least one micropattern of a composite mixture layered on said flexible support, said composite mixture comprising at least a flexible biocompatible polymer and CNTs, wherein said micropattern has a continuous rectangular shape.

14. Process according to claim 13, also comprising a step of calculating the optimal surface area of the micropattern with respect to the surface area of the piezoresistive biosensor by using the following formulae:

$$area\% = \frac{{}^{q}/_{2p} - a_p}{a_s - a_p} \cdot 100$$

15. Process according to any of claims 13 or 14, wherein said process comprises the successive steps of:

- providing a substrate,
- depositing a first flexible biocompatible polymer layer on said substrate,
- preparing a composite mixture by mixing a solution of a second flexible biocompatible polymer and a solution of CNTs,
- depositing a layer of said composite mixture on the surface of said first polymer layer,
- patterning said composite in at least one micropattern, preferably as defined in the calculating step,
- forming metallic electrodes by depositing metal on the opposite ends of said patterned composite layer, and
- removing said substrate, to obtain said piezoresistive biosensor.

16. Process for preparing a biosensor device according to any of claims 10 to 12, comprising a step of calculating the surface area of the micropattern with respect to the surface area of the piezoresistive biosensor by using the following formulae:

$$area\% = \frac{{}^q\!/_{2p} - a_p}{a_s - a_p} \cdot 100$$

and comprising the successive steps of:

- providing a substrate,
- depositing a first layer of a first flexible biocompatible polymer on said substrate,
- depositing a protective material on a first part of the surface of said first polymer layer,
- depositing a second layer of said first flexible biocompatible polymer on a said protective material and on said first layer of said first flexible biocompatible polymer,
- removing said protective material and the first part of said second layer of said first flexible biocompatible polymer,
- depositing a protective material on the second part of said first polymer layer,
- preparing a composite mixture by mixing a solution of a second flexible biocompatible polymer and a solution of CNTs,
- depositing a layer of said composite mixture on the surface of said first polymer layer,
- patterning said composite in at least one micropattern as calculated in the first step,
- removing said protective material,
- forming metallic electrodes by depositing metal on the opposite extremities of said patterned composite layer, and
- depositing of organic FET channel material, and depositing the gate metal,
- removing said substrate, to obtain said biosensor device .

17. Apparatus comprising a piezoresistive biosensor according to any of claims 1 to 9, a biosensor device according to any of claims 10 to 12, a piezoresistive biosensor obtained by a process according to any of claims 13 to 15 or a biosensor device obtained by a process according to claim 16, said apparatus being a biosignal monitoring apparatus chosen among a non-implantable apparatus and an implantable apparatus.

18. Use of at least one apparatus according to claim 17

- for providing a biosignal chosen among: electroencephalogram (EEG), electrocardiogram (ECG), electromyogram (EMG), mechanomyogram (MMG), electrooculography (EOG), galvanic skin response (GSR), magnetoencephalogram (MEG), or
- for monitoring a body parameter chosen among: respiratory flow, body temperature, heart rate and blood pressure.

19. Process for providing a biosignal or for monitoring a body parameter comprising the steps of:

- attaching at least one apparatus according to claim 17 to a surface of said body,
- passing a current through said at least one apparatus, and
- sensing the current passing through said at least one apparatus.

**Figure A**

**Figure B**

**Figure C**

**Fig. 1A to 1C**

$R_{tunneling}$

W

$-p*area+q$

Optimal sensor area

Area optimal threshold

$a_p$

$a_s$

Single pattern area

$q/2p$

Total senso r area

$q/p$

area

**Figure 2**

**Figures 3A and 3B**

**Figure 4**

Figure A   Figure B   Figure C

**Fig. 5A to 5C**

**Figure 6**

**Fig. 7A and 7B**

UV lithography

CNT-PDMS

Substrate

CNT-PDMS deposition

Developed resist

Lift off CNT-PDMS layer

Lift off extra metal

Metal deposition

Final device

Electrode

Electrode

PDMS

Top view of device

**Figure 8**

**Figure 9**

Europäisches
Patentamt

European
Patent Office

Office européen
des brevets

# EUROPEAN SEARCH REPORT

Application Number

EP 18 16 7670

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WENJUN XU ET AL: "Deformable strain sensors based on patterned MWCNTs/polydimethylsiloxane composites", JOURNAL OF POLYMER SCIENCE PART B: POLYMER PHYSICS, vol. 51, no. 20, 15 October 2013 (2013-10-15), pages 1505-1512, XP055499234, US ISSN: 0887-6266, DOI: 10.1002/polb.23361 * page 1506, column first, paragraph second; figure 2a * | 1-19 | INV. A61B5/0408 G01L1/18 |
| X,D | SEUNG MIN LEE ET AL: "Self-adhesive epidermal carbon nanotube electronics for tether-free long-term continuous recording of biosignals", SCIENTIFIC REPORTS, vol. 4, no. 1, 15 August 2014 (2014-08-15), XP055499286, DOI: 10.1038/srep06074 * figure 1f * * page 2, column first * * page 4, column first * | 1-19 | |
| X | US 2016/011063 A1 (ZHANG TING [CN] ET AL) 14 January 2016 (2016-01-14) * paragraphs [0093], [0096], [0097], [0135] - [0166]; figure 1 * | 1-19 | TECHNICAL FIELDS SEARCHED (IPC) A61B G01L |
| X | US 2017/074635 A1 (CHOI JIN-WOO [US] ET AL) 16 March 2017 (2017-03-16) * paragraphs [0018], [0019], [0026]; figure 2H * | 1-19 | |

-/--

The present search report has been drawn up for all claims

| Place of search | Date of completion of the search | Examiner |
|---|---|---|
| The Hague | 13 September 2018 | Chau, Thoi Dai |

EPO FORM 1503 03.82 (P04C01)

page 1 of 2

Europäisches
Patentamt

European
Patent Office

Office européen
des brevets

# EUROPEAN SEARCH REPORT

Application Number

EP 18 16 7670

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | JA HOON KOO ET AL: "Wearable Electrocardiogram Monitor Using Carbon Nanotube Electronics and Color-Tunable Organic Light-Emitting Diodes", ACS NANO, vol. 11, no. 10, 28 August 2017 (2017-08-28), pages 10032-10041, XP055499289, US ISSN: 1936-0851, DOI: 10.1021/acsnano.7b04292 * page 10033; figure 2a * | 1-19 | |

TECHNICAL FIELDS
SEARCHED      (IPC)

The present search report has been drawn up for all claims

| Place of search | Date of completion of the search | Examiner |
|---|---|---|
| The Hague | 13 September 2018 | Chau, Thoi Dai |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 18 16 7670

This annex lists the patent family members relating to the patent documents cited in the above-mentioned European search report.
The members are as contained in the European Patent Office EDP file on
The European Patent Office is in no way liable for these particulars which are merely given for the purpose of information.

13-09-2018

| Patent document cited in search report | | Publication date | Patent family member(s) | | Publication date |
|---|---|---|---|---|---|
| US 2016011063 | A1 | 14-01-2016 | AU | 2014211862 A1 | 10-09-2015 |
| | | | CA | 2899676 A1 | 07-08-2014 |
| | | | EP | 2953012 A1 | 09-12-2015 |
| | | | JP | 6180547 B2 | 16-08-2017 |
| | | | JP | 2016516174 A | 02-06-2016 |
| | | | KR | 20150114509 A | 12-10-2015 |
| | | | US | 2016011063 A1 | 14-01-2016 |
| | | | WO | 2014117724 A1 | 07-08-2014 |
| US 2017074635 | A1 | 16-03-2017 | US | 2012266685 A1 | 25-10-2012 |
| | | | US | 2015302949 A1 | 22-10-2015 |
| | | | US | 2017074635 A1 | 16-03-2017 |
| | | | WO | 2011041507 A1 | 07-04-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8850897 B

- US 9625330 B

### Non-patent literature cited in the description

- **XU ; ALLEN.** Deformable strain sensors based on micropatterned MWCNTs/PDMS composites. *J. Polymer Sci., part B: Polymer Physics,* 2013, vol. 51, 1505-12
- **LEE et al.** Self-adhesive epidermal carbon nanotube electronics for tether-free long-term continuous recording of biosignals. *Nature Scientific Reports,* 2014, vol. 4, 6074

- **FRANKLIN ; CHEN.** Length scaling of carbon nanotube transistors. *Nature Nanotechnology Letters,* 2010, vol. 5, 858-862
- **SO et al.** Carbon nanotube based pressure sensor for flexible electronics. *Nature Nanotechnology Letters,* 2010, vol. 5, 858-862
- **KOO et al.** Wearable electrocardiogram monitor using carbon nanotube electronics and color-tunable organic light-emitting diodes. *ACS NAno,* 2017, vol. 11, 10032-41